# EUROPÄISCHE PATENTSCHRIFT

(11) **EP 1 129 060 B1**
(45) Veröffentlichungstag und Bekanntmachung des Hinweises auf die Patenterteilung: **07.05.2003**
(21) Anmeldenummer: 99971792.9
(22) Anmeldetag: 30.10.1999
(51) Int. Cl.: C07C 47/225, C07C 49/21, C07C 45/51, C11B 9/00, A61K 7/46

(54) **CARBONYLVERBINDUNGEN UND DEREN VERWENDUNG ALS RIECHSTOFFE**
CARBONYL COMPOUNDS AND THEIR USE AS FRAGRANCES
COMPOSES CARBONYLE ET LEUR UTILISATION COMME PARFUMS

(30) Priorität: 10.11.1998 DE 19851684
(43) Veröffentlichungstag der Anmeldung: 05.09.2001
(73) Patentinhaber: Cognis Deutschland GmbH & Co. KG, 40589 Düsseldorf (DE)
(72) Erfinder: MARKERT, Thomas, D-40789 Monheim (DE); LEMKE, Ute, D-41470 Neuss (DE); VEIT, Elke, D-52445 Titz (DE); TEN PIERIK, Theo, NL-5916 Venlo (NL); NEMITZ, Ralph, D-41363 Jüchen (DE); PORRMANN, Volker, D-40723 Hilden (DE); SPEITKAMP, Marc, D-40476 Düsseldorf (DE)
(86) Internationale Anmeldenummer: EP9908282
(87) Internationale Veröffentlichungsnummer: WO00027784

(56) Entgegenhaltungen:
- US-A- 4 069 258

## Beschreibung

### Gebiet der Erfindung

Die Erfindung betrifft neue Carbonylverbindungen spezieller Struktur, ein Verfahren zu deren Herstellung sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, daß die Riechstoffindustrie einen ständigen Bedarf an neuen Riechstoffen und interessanten Duftnoten hat, um einerseits die Palette der natürlich verfügbaren Riechstoffe zu ergänzen und andererseits die notwendigen Anpassungen an wechselnde modische Geschmacksrichtungen vornehmen sowie den ständig steigenden Bedarf an Geruchsverbesserem für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, das heißt angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Mit anderen Worten: Es besteht ein ständiger Bedarf an Verbindungen, die charakteristische neue Geruchsprofile bei gleichzeitig hoher Haftfestigkeit, Geruchsintensität und Strahlkraft aufweisen.

In der US 4,069,258 werden Isopropyl-, bzw. Isopropenyl-substituierte Cyclopenten Derivate und deren Eigenschaften als Riechstoffe beschrieben.

### Beschreibung der Erfindung

Es wurde gefunden, daß die Verbindungen der allgemeinen Formel (I) die obengenannten Forderungen in jeder Hinsicht ausgezeichnet erfüllen und in vorteilhafter Weise als Riechstoffe mit unterschiedlich nuancierten Geruchsnoten mit guter Haftfestigkeit eingesetzt werden können.

Gegenstand der vorliegenden Erfindung sind zunächst Carbonylverbindungen der allgemeinen Struktur (I) 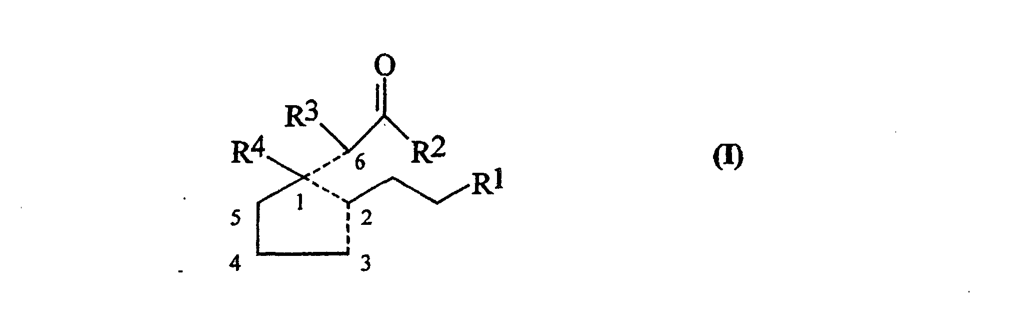 worin der Rest R¹ Wasserstoff oder eine Alkylgruppe mit 1 bis 5 C-Atomen, die Reste R² und R³ unabhängig voneinander Wasserstoff oder eine Alkylgruppe mit 1 bis 3 C-Atomen bedeuten, und in einer der Positionen C-1/C-2, C-2/C-3 oder C-1/C-6 eine C=C-Doppelbindung vorhanden ist und der Rest R⁴ entweder Wasserstoff oder - sofern die C=C-Doppelbindung in der Position C-2/C-3 sitzt - Wasserstoff oder eine Alkylgruppe mit 1 bis 3 C-Atomen bedeuten.

Bevorzugt sind ganz besonders 2-(2'-n-Hexyl-cyclopent-2'-en-1'-yl)acetaldehyd, 2-(2'-n-Hexyl-cyclopent-2'-en-1'-yl)propionaldehyd und (2-n-Hexyl-cyclopent-2-en-1-yl)aceton.

Die Herstellung der Verbindungen (I) kann an sich nach allen dem präparativen Organiker bekannten Methoden erfolgen. Insbesondere geht man jedoch im Rahmen der vorliegenden Erfindung von speziellen Allylalkoholen aus, den 2-Alkyl-cyclopent-2-en-1-olen, die gegebenenfalls in 3-Position eine weitere Alkylgruppe aufweisen können, und die bei der Umsetzung mit speziellen Vinylethem Intermediärprodukte ergeben, die [3,3]-sigmatropen Umlagerungen zugänglich sind. Bei den Umlagerungsprodukten kann sich eine Verschiebung der primär im Fünfring entstandenen C=C-Doppelbindung in Richtung auf die Carbonylgruppe anschließen.

2-Alkyl-cyclopent-2-en-1-one, allgemein unter Bezeichnungen wie Isojasmone, Sedamon, cis-Jasmon, Dijasmon, Dihydrojasmon oder Dihydroisojasmon kommerziell oder durch Aldolkondensation von Cyclopentanon mit diversen Aldehyden erhältliche Produkte, lassen sich mit Hilfe komplexer Hydride wie beispielsweise Alkaliborhydriden oder -aluminiumhydriden oder durch Meerwein-Ponndorf-Reduktion mit Aluminiumalkoholaten selektiv zu den entsprechenden Allylalkoholen, den 2-Alkyl-cyclopent-2-en-1-olen, reduzieren.

Beispiele für geeignete Vinylether sind beispielsweise Ethylvinylether, 2-Methoxypropen, 1-Propenylethylether, 2-Propenylethylether, Cyclohexylvinylether.

Die Verbindungen (I) zeichnen sich geruchlich durch einen ausgeprägt atdehydischen Charakter mit intensiven, grünen und fruchtigen Noten aus.

In Parfüm-Kompositionen verstärken die Verbindungen (I) die Harmonie und Ausstrahlung sowie auch die Haftung, wobei die Dosierung unter Berücksichtigung der übrigen Bestandteile der Komposition auf die jeweils angestrebte Duftnote abgestimmt wird.

Daß die Verbindungen (I) aldehydische, grün-fruchtige Noten aufweisen, war nicht vorhersehbar und ist damit eine weitere Bestätigung für die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahmehmung noch der Einfluß der chemischen Struktur auf die Duftwahrnehmung hinreichend erforscht sind, somit also normalerweise nicht vorhergesehen werden kann, ob ein geänderter Aufbau bekannter Riechstoffe überhaupt zur Änderung der olfaktorischen Eigenschaften führt und ob diese Änderungen positiv oder negativ beurteilt werden.

Ein weiterer Gegenstand der Erfindung ist dementsprechend die Verwendung der Verbindungen (I) als Riechstoffe.

Die Verbindungen der Formel (I) eignen sich aufgrund ihres Geruchsprofils insbesondere auch zur Modifizierung und Verstärkung bekannter Kompositionen. Hervorgehoben werden soll insbesondere ihre außerordentliche Geruchsstärke, die ganz allgemein zur Veredelung der Komposition beiträgt.

Die Verbindungen der Formel (I) lassen sich mit zahlreichen bekannten Riechstoffingredientien, z.B. anderen Riechstoffen natürlichen, synthetischen oder partial-synthetischen Ursprungs, etherischen Ölen und Pflanzenextrakten kombinieren. Die Palette der natürlichen Riechstoffe kann dabei sowohl leicht- als auch mittel- und schwerflüchtige Komponenten und diejenige der synthetischen Riechstoffe Vertreter aus praktisch allen Stoffklassen umfassen. Beispiele sind:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl, Myrrheöl, Olibanumöl,
(b) Alkohole wie Famesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional^{R}, α-Hexylzimtaldehyd, Hydroxycitronellal, Lilial^{R} [p-tert.-Butyl-α-methyldihydrozimtaldehyd], Methyinonylacetaldehyd,
(d) Ketone wie Allylionon, α-lonon, β-lonon, Isoraldein, Methylionon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,
(f) Lactone wie γ-Undecalacton, 1-Oxaspiro[4.4]nonan-2-on,
sowie verschiedene weitere in der Parfümerie oft benutzte Komponenten wie Moschus, Indol, p-Menthan-8-thiol-3-on, Methyleugenol, Ambroxan.

Bemerkenswert ist ferner die Art und Weise, wie die Verbindungen der Struktur (I) die Geruchsnoten einer breiten Palette bekannter Kompositionen abrunden und harmonisieren, ohne aber in unangenehmer Weise zu dominieren. 2-(2'-n-Hexyl-cyclopent-2'-en-1'-yl)antaldehyd ist in dieser Hinsicht ganz besonders hervorzuheben.

Die erfindungsgemäßen Verbindungen enthalten Chiralitätszentren, so daß diese Verbindungen in verschiedenen Raumformen existieren können. Im Rahmen üblicher Synthesen fallen die erfindungsgemäßen Verbindungen als Gemische der entsprechenden Isomeren an und werden als solche als Riechstoffe verwendet.

Die einsetzbaren Anteile der erfindungsgemäßen Verbindungen oder deren Gemische in Riechstoffkompositionen bewegen sich von 1 bis 70 Gewichtsprozent, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Puder, Aerosole, Zahnpasten, Mundwässer, Desodorantien als auch in der alkoholischen Parfümerie (z.B. Eaux de Cologne, Eaux de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 bis 2 Gewichtsprozent, bezogen auf das gesamte Produkt, zugesetzt. Diese Werte sollen jedoch keine Grenzwerte darstellen, da der erfahrene Parfümeur auch mit noch geringeren Konzentrationen Effekte erzielen oder aber mit noch höheren Dosierungen neuartige Komplexe aufbauen kann.

### Beispiele

### A) Vorstufen

### Beispiel 1

### Darstellung von "Isojasmon"

### (2-n-Hexyl-2-cyclopenten-1-on und 2-n-Hexylidencyclopentan-1-on)

| | |
|---|---|
| Ansatz | 210 g (2,5 Mol) Cyclopentanon |
| | 280 g (2,8 Mol) n-Hexanal |
| | 280 ml (0,25 Mol) KOH-Lösung 5%ig |
| | 280 ml Cyclohexan |
| | 300 g (0,3 Mol) Schwefelsäure 5%ig |
| | 0,17 g p-Toluolsulfonsäure |

Ausführung: Die Reaktion wurde in einem 2-I-Planschliffkolben mit eingebauten Schikanen und einem Turbinenrührer (1000 U/min) durchgeführt. Die 5%ige KOH und das Cyclohexan wurden unter Stickstoff bei 15°C vorgelegt. Unter Rühren wurde die Lösung aus Cyclopentanon und Hexanal bei 15 - 20°C in 2 Stunden zugetropft. Man ließ noch 2 Stunden nachrühren und neutralisierte dann mit der Schwefelsäure. Der Ansatz wurde in einen Scheidetrichter überführt und die Phasen wurden getrennt. Die wäßrige Phase wurde verworfen und die Cyclohexanphase wurde mit dest. Wasser neutral gewaschen. Ohne die organische Phase zu trocknen wurde diese mit der p-Toluolsulfonsäure versetzt und zur azeotropen Abdestillation von ca. 50 ml Wasser zum Rückfluß am Wasserabscheider erhitzt.
Es wurden weitere 0,8 g p-Toluolsulfonsäure zugegeben und noch einmal 7 ml Wasser ausgekreist.

Aufarbeitung: Das Cyclohexan wurde abdestilliert und die 400 g schwarzer Rückstand wurden über einen mit Öl beheizten Dünnschichtverdampfer (Manteltemperatur 115-120°C/0,1 mbar) destilliert.

Bei der anschließenden fraktionierten Destillation an einer Drehbandkolonne wurde das Produkt als Isomerengemisch (Verbindungen mit exo- und endozyklischer Doppelbindung) in einer Ausbeute von 45 % der Theorie isoliert.

### Beispiel 2

### Isomerisierung des 2-n-Hexylidencyclopentanons zu 2-n-Hexyl-cyclopent-2-en-1-on

### [gemäß J.G.Tillett, Chem.Rev. 76, 747 (1976)]

| | |
|---|---|
| Ansatz | 332 g (2 Mol) Isojasmon (Isomerengemisch aus Beispiel 1) |
| | 2 I 1-Butanol |
| | 0,2 I konz. Salzsäure |

Ausführung: In einem 4-I-Dreihalskolben mit KPG-Rührer, Tropftrichter, Intensivkühler und Temperatursonde wurden das Isojasmon und n-Butanol vorgelegt und bei Raumtemperatur tropfenweise mit der Salzsäure versetzt. Es war ein leichter Temperaturanstieg zu verzeichnen. Der Ansatz wurde noch 3 Stunden bei ca. 100°C gerührt und anschließend mit Diethylether verdünnt.

Aufarbeitung: Die organische Phase wurde abgetrennt und mit Wasser, Bicarbonat-Lösung und Wasser ausgewaschen. Nach Trocknen über Magnesiumsulfat wurde filtriert und eingeengt Es wurden 329,5 g Rückstand ausgewogen. Das Material wurde an einer 10 cm Vigreux-Kolonne im Hochvakuum destilliert, wobei 171,9 g 2-n-Hexyl-cyclopent-2-en-1-on mit über 99%iger Reinheit gewonnen werden konnten. Die Ausbeute betrug 51,8% der Theorie.

### Beispiel 3

### Reduktion von Isojasmon zu Isojasmol

### (gemäß J.L.Luche, L.Rodriguez-Hahn, P.Grabbe, J.C.S. Chem.Comm., 1978, S.601-2)

| | | |
|---|---|---|
| Ansatz | I) | 167,8 g (1 mol) Isojasmon (hergestellt nach Bsp. 2 oder handelsübliches Produkt, etwa von Firma Quest) |
| | II) | 373 g (1 mol) Cer(III)chloridheptahydrat |
| | III) | 38 g Natriumborhydrid |
| | IV) | 1,5 l Methanol |

Die Komponenten I, II und IV wurden in einem 4-I-Vierhalskolben bei Raumtemperatur gemischt. Die Komponente III wurde innerhalb von 3 Stunden unter starkem Rühren portionsweise zugegeben. Es wurde 2 Stunden bei Raumtemperatur nachgerührt.

Aufarbeitung: Dem Ansatz wurden ca. 1 Liter Wasser zugesetzt und anschließend wurde 4 mal mit Cyclohexan extrahiert Die organische Phase wurde über Natriumsulfat getrocknet und am Rotationsverdampfer eingeengt.

Der Rückstand von 161,3 g Reaktionsgemisch wurde an einer 15 cm Vigreuxkolonne destilliert. Es wurden 136,1 g Produkt mit einer gaschromatographisch bestimmten Reinheit von ca. 90% (73% der Theorie) erhalten, die ohne weitere Reinigung für nachfolgende Synthesen eingesetzt wurden.

### Beispiel 4

### Herstellung von 3-Methyl-2-n-heptyl-cyclopent-2-en-1-on ausgehend vom Henkel-Riechstoff "Aldehyd 11/11"

| | |
|---|---|
| Ansatz | 170 g (1 mol) Aldehyd 11/11 (Gemisch aus Methyloctylacetaldehyd und Undecanal) |
| | 125 g (1,2 mol) Malonsäure |
| | 4 g Ammoniumacetat |
| | 400 ml Toluol |

Ausführung: In einem 1-I-Dreihalskolben mit Rührer, Innenthermometer und Dean-Starck-Wasserabscheider wurden alle Komponenten schnell vereinigt und unter starkem Rühren zum Rückfluß gebracht. Nach 3 Stunden waren 18 ml Wasser azeotrop abdestilliert und abgetrennt worden. Anschließend wurden bei 50°C Ölbadtemperatur im Wasserstrahlvakuum ca. 280 g Toluol abdestilliert und der verbleibende Rückstand an einer Kurzwegdestille (Manteltemperatur 205°C, 0.03 mbar) destilliert. Dabei wurden 115 g (54,3% der Theorie) 4-Methyl-dodec-3-ensäure erhalten.

Die 115 g 4-Methyl-dodec-3-ensäure wurden in einem 500 ml Dreihalskolben unter Rühren mit 115 g 85%iger Schwefelsäure versetzt und die Mischung wurde 4 Stunden auf 90°C erhitzt. Zur Aufarbeitung wurde das Reaktionsgemisch vorsichtig in 500 ml Wasser eingerührt, 3 mal mit 250 ml Ether extrahiert und die vereinigten Etherphasen wurden mit Bicarbonat-Lösung neutral gewaschen, über Natriumsulfat getrocknet und am Rotavapor eingeengt. Der Rückstand von 92 g wurde an einer Kugelrohrdestille bei 180°C und 0.03 mbar Druck destilliert, wobei 66 g (57,4% der Theorie) 5-Methyl-5-n-octylbutyrolacton und 5 g Rückstand erhalten wurden.

In einem 1-I-Dreihalskolben wurden 200 g Polyphosphorsäure (Fa. Fluka) unter Stickstoff und Rühren auf 100°C erhitzt und 63,6 g (0.3 mol) 5-Methyl-5-n-octylbutyrolacton in 45 Minuten zugetropft. Die Mischung wurde 1,5 Stunden bei 100°C gerührt und danach abgekühlt. Das Produkt wurde mit 3 mal 150 ml Diethylether extrahiert, mit Bicarbonat-Lösung neutral gewaschen, über Natriumsulfat getrocknet und am Rotavapor eingeengt. 60 g Rückstand (GC-Reinheit: 78%) wurden an einer Kugelrohrdestille (Ofentemperatur 150°C, 0.03 mbar) vordestilliert. Die dabei erhaltenen 58 g wurden an einer 15 cm langen Vigreuxkolonne destilliert, der Hauptlauf von 32,5 g, dessen Reinheit 95% betrug, wurde an einer Drehbandkolonne fraktioniert. Dabei wurden 20,8 g 3-Methyl-2-n-heptyl-2-cyclopenten-1-on (Sdp. 74-76°C/0.08 mbar) in 100%iger Reinheit erhalten.
Geruchsbeschreibung: Jasmon, diffusiv, blumig, Sellerie-Note

### B) Erfindungsgemäße Verbindungen

### Beispiel 5

### Umsetzung von Isojasmol zu 2-(2'-n-Hexyl-cyclopent-2'-en-1'-yl)acetaldehyd

| | |
|---|---|
| Ansatz | 433,1 g (2 mol) Isojasmol (hergestellt nach Bsp.3) |
| | 173,1 g (2,4 mol) Ethylvinylether (99%, Fa. Fluka) |
| | 18 g (0,24 mol) Propionsäure |

Ausführung: Die Komponenten wurden unter Stickstoff in einen 2-I-V2a-Stahlautoklaveneinsatz gefüllt und in einem Autoklaven unter 50 bar Stickstoff bei 180°C 4 Stunden gerührt. Anschließend wurden erneut 18 g Propionsäure zu der Reaktionsmischung gegeben und unter 60 bar Stickstoff bei 200°C erneut 4 Stunden gerührt. Wie man über GC leicht feststellen konnte, entstanden bei 180°C eine Reihe von Zwischenprodukten, die sich bei 200°C zum Teil in das Edukt Isojasmol und zum Teil in das gewünschte Produkt (2 Hauptkomponenten) umsetzten.

Aufarbeitung: Nach Abdestillieren von Leichtsiedem wurden 459,2 g Rohprodukt zur Destillation im Hochvakuum an einer Drehbandkolonne eingesetzt. 79 g Hauptlauf gingen bei Kopftemperaturen von 64 - 73°C/0.04 mbar über (2 Isomere; 60 % im GC). Diese niedrige Reinheit war geruchlich akzeptabel. Geruchsbeschreibung: aldehydisch, grün, Ozon, blumig, fruchtig, Wassermelone, fettig.

### Beispiel 6

### Umsetzung von Isojasmol zu 2-(2'-n-Hexyl-cyclopent-2'-en-1'-yl)-propionaldehyd

| | |
|---|---|
| Ansatz | 84,0 g (0,5 mol) Isojasmol (hergestellt nach Bsp. 3) |
| | 56,0 g (0,65 moi) Ethyl-1-propenylether (Fluka) |
| | 1,5 g Propionsäure (Merck) |

Ausführung: Analog Beispiel 5 wurden die Komponenten in einem V2a-Stahlautoklaveneinsatz vorgelegt und unter 10 bar Stickstoff-Anfangsdruck auf 210°C erwärmt und 8 Stunden rühren gelassen. Der Druck stieg dabei auf 30 bar an.
Aufarbeitung: Das Rohprodukt wurde am Rotavapor von überschüssigem Ether befreit und 95,4 g dunkelbraune Flüssigkeit wurden im Hochvakuum über eine 30 cm-Füllkörperkolonne (Braunschweiger Wendeln) destilliert. Der Hauptlauf von 24,5 g hellgelbe Flüssigkeit (Sdp. 62 - 93°C/0.08 mbar) wurde an einer Drehbandkolonne fraktioniert. Dabei wurden 15,4 g 2-(2-n-Hexylcyclopent-2-en-1-yl)-propionaldehyd (Sdp. 60-64°C/0.04 mbar) erhalten. Die gaschromatographisch bestimmte Reinheit betrug 93%.

Geruchsbeschreibung: grün, aldehydisch, blumig, strahlend (diffusiv), fruchtig, Melone, Ozon.

### Beispiel 7

### Umsetzung von Isojasmol zu (2-n-Hexylcyclopent-2-en-1-yl)aceton

| | |
|---|---|
| Ansatz | 84,0 g (0,5 mol) Isojasmol (hergestellt nach Bsp. 3) |
| | 56,0 g (0,78 mol) 2-Methoxypropen (Fa. Janssen) |
| | 1,5 g Propionsäure (Merck) |

Ausführung: Die Komponenten wurden in einem V2a-Stahlautoklaveneinsatz unter 10 bar Stickstoff vorgelegt und 8 Stunden unter Rühren auf 210°C erhitzt (30 bar Arbeitsdruck).

Aufarbeitung: Das Rohprodukt wurde von überschüssigem Ether befreit und der Rückstand, 85,8 g dunkelbraune Flüssigkeit, wurde zur Destillation im Hochvakuum an einer 30 cm langen, mit Braunschweiger Wendeln gefüllten, Kolonne eingesetzt.
Als Hauptlauf wurden 37,3 g hellgelbe Flüssigkeit vom Sdp. 80 -94°C/0.08 mbar erhalten, die an einer Drehbandkolonne fraktioniert wurden.

Es wurden 33,4 g hellgrüne Flüssigkeit mit Sdp. 85 - 87°C/0.06 mbar erhalten.
Geruchsbeschreibung: blumig, diffusiv, würzig, Sellerie-Note, pilzig.

### Beispiel 8

### Darstellung von (2'-n-Hexyl-cyclopentyliden)-acetaldehyd ausgehend von 2-n-Hexyl-cyclopentan-1-on

### Stufe 1

| | |
|---|---|
| Ansatz | 83,9 g (0.5 mol) Jasmatone (2-n-Hexyl-cyclopentan-1-on; Fa. Quest) |
| | 111,0 g (0,75 mol) Triethylorthoformiat |
| | 101,8 mg Kaliumhydrogensulfat |
| | 500 ml Ethanol, wasserfrei |

Apparatur: 1-I-Rührapparatur, KPG-Rührführung, Thermometer, Rückflußkühler

Ausführung: Die Apparatur wurde unter Durchleiten eines konstanten Stickstoffstroms (25 ml/min) mit einem Heißluftföhn getrocknet und ausgeheizt. Das Jasmatone wurde in 200 ml abs. Ethanol vorgelegt, danach wurden das Triethylorthoformiat, das Kaliumhydrogensulfat und die restlichen 300 ml Ethanol zugegeben. Die Abnahme an Edukt und die Bildung von mehreren Produkten wurde über GC verfolgt. Nach 5 Stunden wurde die Reaktion abgebrochen und der Ansatz durch Zugabe einer kleinen Menge Natriumethanolat-Lösung neutralisiert. Der Ansatz wurde mit 2 weiteren Ansätzen, die unter denselben Bedingungen durchgeführt worden waren, vereinigt und die insgesamt 411,5 g Rohprodukt wurden an einer 20 cm langen Vigreuxkolonne destilliert. Dabei wurden als Hauptlauf 252,3 g Jasmatonediethylketal (1,1-Diethoxy-2-n-hexylcyclopentan) (GC-Reinheit: 97% (2 Peaks), Sdp. 95-98°C/0.08 mbar) erhalten.

### Stufe 2

| | |
|---|---|
| Ansatz | 331,2 g (1,34 mol) 1,1-Diethoxy-2-n-hexyl-cyclopentan (hergestellt nach Stufe 1) |
| | 144,9 g (2 mol) Ethylvinylether |
| | 115 ml Zink(II)chloridlösung, 10%ig in Essigsäureethylester |

Apparatur: 1-I-Rührapparatur, Rückflußkühler, Tropftrichter, Thermometer

Ausführung: Das 1,1-Diethoxy-2-n-hexyl-cyclopentanon und die Zinkchloridlösung wurden unter Rühren vorgelegt und auf 40 - 45°C erwärmt. In 2 Stunden wurde der Ethylvinylether kontinuierlich zu diesem Gemisch zudosiert. Nach voliendeter Zugabe wurde 3 Stunden bei 40 - 45°C gerührt.

Aufarbeitung: Das Reaktionsgemisch wurde 2 mal mit 0,1 molarer Natronlauge und 2 mal mit Wasser gewaschen, über Natriumsulfat getrocknet und am Rotavapor eingeengt. Die Destillation an einer 20 cm langen Vigreuxkolonne ergab als Hauptlauf 61,5 g ca. 62%iges 1-(2,2-Diethoxy-ethyl)-1-ethoxy-2-hexyl-cyclopentan (2 Diastereomere, Sdp. 130 - 140°C/0.04 mbar).

### Stufe 3

| | |
|---|---|
| Ansatz | 61,5 g (0,121 mol) 1-(2,2-Diethoxy-ethyl)-1-ethoxy-2-hexyl-cyclopentan (hergestellt nach Stufe 2) |
| | 18,7 g (0,41 mol) Ameisensäure |
| | 5,0 g (0,074 mol) Natriumformiat |
| | 8,0 g (0,443 mol) Wasser, entmineralisiert |

Apparatur: 250 ml Rührapparatur, PT 100 (Temperaturmeßfühler aus Edelstahl), Rückflußkühler, Tropftrichter.
Ausführung: Der Ameisensäure/Natriumformiat-Puffer wurde vorgelegt und auf Rückflußtemperatur (113°C) erwärmt. Die 61,5 g 1-(2,2-Diethoxy-ethyl)-1-ethoxy-2-hexyl-cyclopentan wurden bei Rückflußtemperatur innerhalb 1,15 Stunden unter Rühren kontinuierlich zudosiert. Die Mischung wurde noch 2 Stunden bei Rückflußtemperatur (jetzt 78°C) gehalten.

Aufarbeitung: Der Ansatz wurde in 200 ml Eiswasser eingerührt und die organische Phase abgetrennt. Danach wurde die wäßrige Phase 3 mal mit je 100 ml Diethylether extrahiert und die vereinigten organischen Phasen neutral gewaschen, über Magnesiumsulfat getrocknet und am Rotavapor eingeengt. Die Fraktionierung von 55 g Rohprodukt an einer Drehbandkolonne ergab Produktfraktionen unterschiedlicher GC-Reinheiten (Gesamtmenge der Fraktionen: 38,5 g).

Fraktion 3 mit einer Zusammensetzung von 49/15/19 GC% (Sdp. 63 - 82°C/0.08 mbar; 2-n-Hexylcyclopentylidenacetaldehyd und Isomere mit Doppelbindung in 1- oder 2-Stellung) wurde geruchlich als vergleichbar mit dem Geruch der Verbindung gemäß Beispiel 6 beurteilt: grün, aldehydisch, frisch, weniger Melone und Ozon als in Beispiel 6.

Bei den weiteren Fraktionen trat zusätzlich noch eine fettige Note auf.

## Patentansprüche

1. Carbonylverbindungen der allgemeinen Struktur (I) 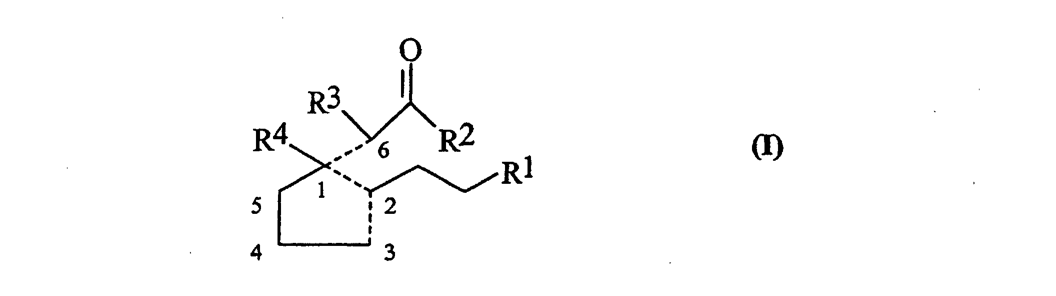 worin der Rest R¹ Wasserstoff oder eine Alkylgruppe mit 1 bis 5 C-Atomen, die Reste R² und R³ unabhängig voneinander Wasserstoff oder eine Alkylgruppe mit 1 bis 3 C-Atomen bedeuten, und in einer der Positionen C-1/C-2, C-2/C-3 oder C-1/C-6 eine C=C-Doppelbindung vorhanden ist und der Rest R⁴ entweder Wasserstoff oder - sofern die C=C-Doppelbindung in der Position C-2/C-3 sitzt - Wasserstoff oder eine Alkylgruppe mit 1 bis 3 C-Atomen bedeuten.

2. 2-(2'-n-Hexyl-cyclopent-2'-en-1'-yl)acetaldehyd.

3. 2-(2'-n-Hexyl-cyclopent-2'-en-1'-yl)propionaldehyd.

4. (2-n-Hexyl-cyclopent-2-en-1-yl)aceton.

5. Verfahren zur Herstellung von Carbonylverbindungen der allgemeinen Struktur (I) 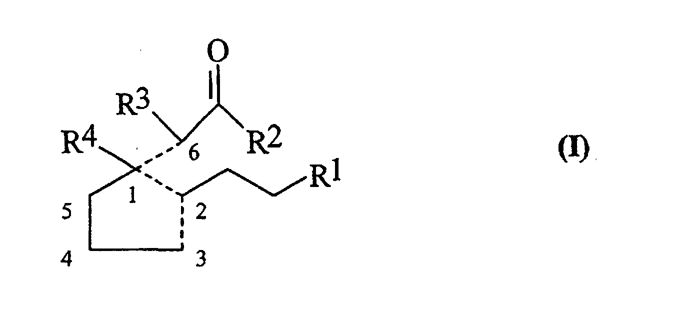 worin der Rest R¹ Wasserstoff oder eine Alkylgruppe mit 1 bis 5 C-Atomen, die Reste R² und R³ unabhängig voneinander Wasserstoff oder eine Alkylgruppe mit 1 bis 3 C-Atomen bedeuten, und in einer der Positionen C-1/C-2, C-2/C-3 oder C-1/C-6 eine C=C-Doppelbindung vorhanden ist und der Rest R⁴ entweder Wasserstoff oder - sofern die C=C-Doppelbindung in der Position C-2/C-3 sitzt - Wasserstoff oder eine Alkylgruppe mit 1 bis 3 C-Atomen bedeuten, wobei man die entsprechenden 2-Alkylcyclopent-2-en-1-ole, die gegebenenfalls in 3-Position eine weitere Alkylgruppe aufweisen können, mit dem entsprechenden Vinylethern umsetzt.

6. Verwendung von Carbonylverbindungen der allgemeinen Struktur (I) 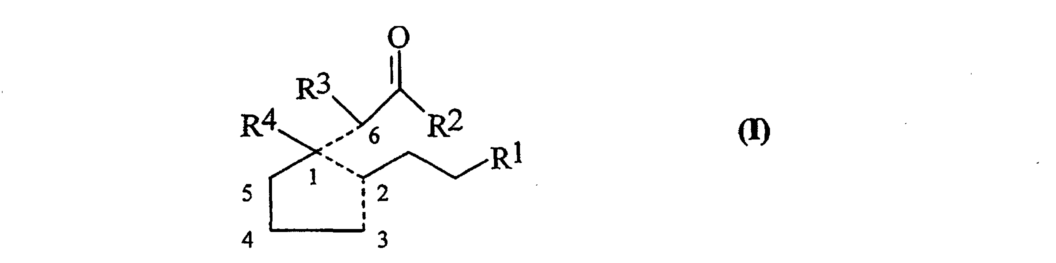 worin der Rest R¹ Wasserstoff oder eine Alkylgruppe mit 1 bis 5 C-Atomen, die Reste R² und R³ unabhängig voneinander Wasserstoff oder eine und der Rest R⁴ entweder Wasserstoff oder - sofern die C=C-Doppelbindung in der Position C-2/C-3 sitzt - Wasserstoff oder eine Alkylgruppe mit 1 bis 3 C-Atomen bedeuten, als Riechstoffe.

7. Riechstoffkompositionen mit einem Gehalt an einem oder mehreren Carbonylverbindungen (I) gemäß Anspruch 1 in einer Menge von 1 - 70 Gew.-% (bezogen auf die gesamte Komposition).

## Claims

1. Carbonyl compounds corresponding to general formula (I): 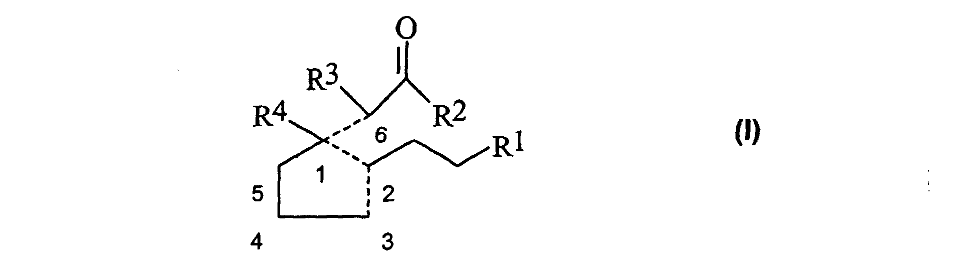 in which R¹ is hydrogen or a C₁₋₅ alkyl group, R² and R³ independently of one another represent hydrogen or a C₁₋₃ alkyl group, a C=C double bond is present in one of the positions C-1/C-2, C-2/C-3 or C-1/C-6 and R⁴ is either hydrogen or - if the C=C double bond is in the C-2/C-3 position - is hydrogen or a C₁₋₃ alkyl group.

2. 2-(2'-n-hexylcyclopent-2'-en-1'-yl)-acetaldehyde.

3. 2-(2'-n-hexylcyclopent-2'-en-1'-yl)-propionaldehyde.

4. (2-n-hexylcyclopent-2-en-1-yl)-acetone.

5. A process for the production of carbonyl compounds corresponding to general formula (I): 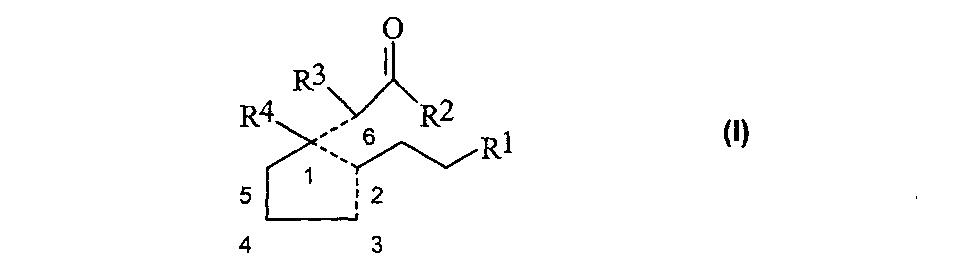 in which R¹ is hydrogen or a C₁₋₅ alkyl group, R² and R³ independently of one another represent hydrogen or a C₁₋₃ alkyl group, a C=C double bond is present in one of the positions C-1/C-2, C-2/C-3 or C-1/C-6 and R⁴ is either hydrogen or - if the C=C double bond is in the C-2/C-3 position - is hydrogen or a C₁₋₃ alkyl group,
**characterized in that** the corresponding 2-alkylcyclopent-2-en-1-ols, which may optionally contain another alkyl group in the 3-position, are reacted with the corresponding vinyl ethers.

6. The use of carbonyl compounds corresponding to general formula (I): 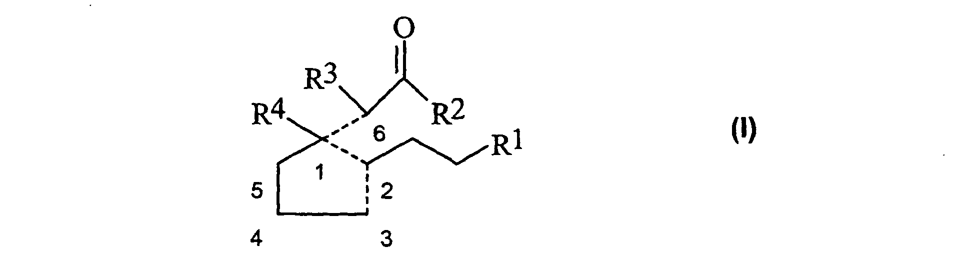 in which R¹ is hydrogen or a C₁₋₅ alkyl group, R² and R³ independently of one another represent hydrogen or a C₁₋₃ alkyl group, a C=C double bond is present in one of the positions C-1/C-2, C-2/C-3 or C-1/C-6 and R⁴ is either hydrogen or - if the C=C double bond is in the C-2/C-3 position - is hydrogen or a C₁₋₃ alkyl group,
as perfumes.

7. Perfume compositions containing one or more of the carbonyl compounds (I) claimed in claim 1 in a quantity of 1 to 70% by weight (based on the composition as a whole).

## Revendications

1. Dérivés carbonylés de structure générale : 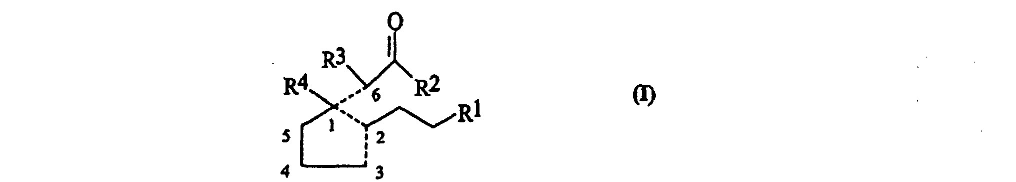 dans laquelle le reste R¹ signifie de l'hydrogène ou un groupe alkyle ayant de 1 à 5 atomes de carbone, les restes R² et R³ indépendamment l'un de l'autre signifient de l'hydrogène ou un groupe alkyle ayant de 1 à 3 atomes de carbone et dans laquelle une double liaison C=C est présente en l'une des positions C-1/C-2, C-2/C-3 ou C-1/C-6, et le reste R⁴ signifie, soit de l'hydrogène soit pour autant que la double liaison C=C se situe en position C-2/C-3, de l'hydrogène ou un groupe alkyle ayant de 1 à 3 atomes de carbone.

2. 2-(2'-n-hexyl-cyclopenta-2'-en-1'-yl)acétaldéhyde.

3. 2-(2'-n-hexyl-cyclopenta-2'-en-1'-yl)propionaldéhyde.

4. (2-n-hexyl-cyclopenta-2-en-1-yl)acétone.

5. Procédé de préparation de composés carbonylés de structure générale (I), 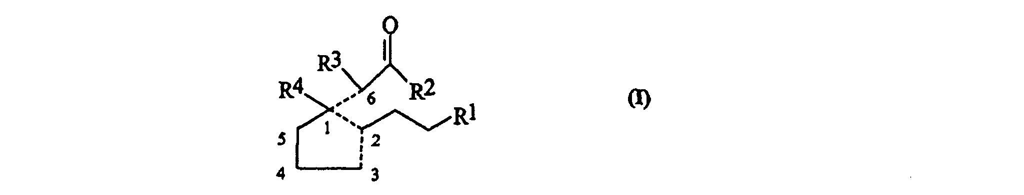 dans laquelle :
le reste R¹ signifie de l'hydrogène ou un groupe alkyle ayant de 1 à 5 atomes de carbone, les restes R² et R³ indépendamment l'un de l'autre signifient de l'hydrogène ou un groupe alkyle ayant de 1 à 3 atomes de carbone et en une des positions C-1/C-2, C-2/C-3, ou C-1/C-6 une double liaison C=C est présente, et le reste R⁴ signifie soit de l'hydrogène soit, pour autant que la double liaison C=C est située dans la position C-2/C-3, de l'hydrogène ou un groupe alkyle ayant de 1 à 3 atomes de carbone, dans lequel on fait réagir les 2-alkyl-cyclopenta-2-en-1-oles, correspondants, qui peuvent posséder le cas échéant en position 3, un autre groupe alkyle, avec l'éther vinylique correspondant.

6. Utilisation des composés carbonylés de structure générale (I), 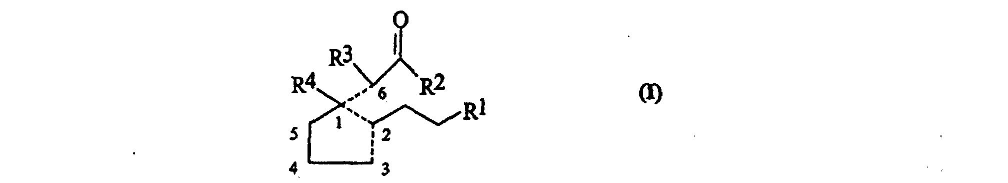 dans laquelle,
R¹ signifie de l'hydrogène ou un groupe alkyle ayant de 1 à 5 atomes de carbone, les restes R² et R³ indépendamment l'un de l'autre signifient de l'hydrogène ou un groupe alkyle ayant de 1 à 3 atomes de carbone, le reste R⁴ signifie de l'hydrogène ou pour autant que la double liaison soit située en position C-2/C-3 de l'hydrogène ou un groupe alkyle ayant de 1 à 3 atomes de carbone en tant que substances odorantes.

7. Compositions de substance odorante ayant une teneur en un ou plusieurs composés carbonylés (I) conformément à la revendication 1, en une quantité de 1 à 70 % en poids (rapporté à la composition totale).
